# EUROPEAN PATENT APPLICATION

(11) **EP 2 236 113 A1**
(43) Date of publication of application: **06.10.2010**
(21) Application number: 08862440.8
(22) Date of filing: 18.08.2008
(51) Int. Cl.: A61F 13/49, A61F 13/15, A61F 13/494, A61F 13/511

(54) **ABSORBENT ARTICLE**

(30) Priority: 18.12.2007 JP 2007326314
(71) Applicant: Uni-charm Corporation, Ehime 799-0111 (JP)
(72) Inventor: MINATO, Hironao, Kanonji-shi Kagawa 769-1602 (JP); NAKAJIMA, Kaiyo, Kanonji-shi Kagawa 769-1602 (JP); KAMIYAMA, Ryuichi, Kanonji-shi Kagawa 769-1602 (JP); NITTA, Reiko, Kanonji-shi Kagawa 769-1602 (JP)
(74) Representative: Stark, Gordon Drummond
(86) International application number: PCT/JP2008/064693
(87) International publication number: WO 2009/078197

(57) **Abstract**

The present invention aims to provide an absorbent article to prevent stuffiness generated between a barrier sheet and a wearer's skin and thereby to prevent eczema due to such stuffiness.

An absorbent article 1 including a chassis 2 and a barrier sheet 3. The barrier sheet has a pair of lateral zones 30, 31 opposed to each other in a transverse direction and a middle zone 32 connecting the lateral zones and the middle zones lies on a crotch region. Front and rear through-holes 33, 34 are defined by the lateral zones and the middle zone. The lateral zones lying outside the middle zone as viewed in the transverse direction are partially cutaway to form notches 41. The notches are formed by the lateral zones.

## Description

### TECHNICAL FIELD

The present invention relates to absorbent articles and more particularly to absorbent articles such as disposable diapers, toilet-training pants or incontinent briefs.

### RELATED ART

Disposable diapers provided with skin-contact sheets serving to protect the wearers' skin from contact with body waste are known, for example, from the disclosure of JP2007-105298A (PATENT DOCUMENT 1). According to the disclosure of PATENT DOCUMENT 1, the diaper comprises a topsheet, a backsheet, an absorbent structure sandwiched between these topsheet and backsheet and a skin-contact sheet disposed on the side of the topsheet facing a wearer's skin wherein the skin-contact sheet is provided with at least one through-hole adapted to guide body waste to the side of the topsheet and elastic members attached under tension to the skin-contact sheet so as to surround the through-hole.
PATENT DOCUMENT 1: JP2007-105298A

### DISCLOSURE OF THE INVENTION

### PROBLEM TO BE SOLVED BY THE INVENTION

The diaper disclosed in PATENT DOCUMENT 1 uses water-repellent as material for the skin-contact sheet to prevent the skin contact sheet from being wetted with body waste. Such skin contact sheet is lifted up under the effect of the elastic members and always held in contact with a wearer's skin. Contact of such water-repellent skin contact sheet with a wearer's skin for a long time causes stuffiness due to sweat of the wearer and may cause eczema.

In view of the problem as has been described just above, it is a principal object of the invention to prevent stuffiness generated between the barrier sheet and a wearer's skin and thereby to prevent eczema due to such stuffiness.

### MEASURE TO SOLVE THE PROBLEM

The invention relates to an improvement in the absorbent article comprising a chassis having a front-to-rear direction and a transverse direction, sides facing a wearer's skin and wearer's clothes, respectively, a front waist region, a rear waist region and a crotch region extending between the front and rear waist regions, and a liquid-absorbent structure disposed on the crotch region, and a barrier sheet lying on the side of the chassis facing a wearer's skin and provided in the crotch region so as to be spaced from the chassis.

The absorbent article according to the invention is **characterized in that** the barrier sheet has a pair of lateral zones extending in the front-to-rear direction and opposed to each other in the transverse direction, a middle zone connecting the lateral zones to each other, front and rear ends extending in the transverse direction and opposed to each other in the front-to-rear direction and barrier sheet's elastic members extending in the front-to-rear direction and opposed to each other in the transverse direction and attached under tension to the barrier sheet wherein the front and rear ends are permanently bonded to the side of chassis facing a wearer's skin and the liquid-absorbent structure is adapted to be bowed in a direction from the crotch region toward the front and rear waist regions and thereby to be spaced from the chassis and form a pocket between the barrier sheet and the liquid-absorbent structure, through-holes are defined by the pair of lateral zones and the middle zone so that body waste can pass the through-holes into the pocket, and the pair of lateral zones lying outside the middle zones as viewed in the transverse direction are partially cutaway to form notches.

According to another preferred embodiment, the notches are formed by cutting off the lateral zones partially inclusive of side edges wherein the side edges are forming the outside of the pair of lateral zones as viewed in transverse direction.

According to another preferred embodiment, the notches are formed by cutting out the lateral zones with side edges remaining wherein the side edges are forming outside of the pair of lateral zones as viewed in transverse direction.

According to another preferred embodiment, the through-hole comprises the front through-hole lying on the front side with respect to the middle zone in the front-to-rear direction and the rear through-hole lying on the rear side with respect to the middle in the front-to-rear direction, the barrier sheet includes the border line bisecting the middle zone in the front-to-rear direction, the notches are formed about the border line and divided by this border line into the front and rear zones in the front-to-rear direction wherein the front zone has an area larger than the rear zone.

According to another preferred embodiment, there is provided between the barrier sheet and the chassis the partition sheet extending from the middle zone to the notches in the transverse direction and adapted to partition the pocket into the side of the front waist region and the side of the rear waist region.

The absorbent article according to the invention is further **characterized in that**
the barrier sheet has a pair of lateral zones extending in the front-to-rear direction and opposed to each other in the transverse direction, a middle zone connecting the lateral zones to each other, front and rear ends extending in the transverse direction and opposed to each other in the front-to-rear direction and barrier sheet's elastic members extending in the front-to-rear direction and attached under tension to the barrier sheet and opposed to each other in the transverse direction wherein the front and rear ends are permanently bonded to the side of the chassis facing a wearer's skin and the liquid-absorbent structure is adapted to be bowed in a direction from the crotch region toward the front and rear waist regions and thereby to be spaced from the chassis and form a pocket between the barrier sheet and the liquid-absorbent structure, through-holes are defined by the pair of lateral zones and the middle zone so that body waste can pass the through-holes into the pocket, the lateral zones lying outside said middle zones as viewed in the transverse direction are provided with middle elastic members attached under tension thereto so as to extend in the transverse direction wherein, outside of these middle elastic members as viewed in the transverse direction, the barrier sheet and the chassis are not bonded to each other.

According to another preferred embodiment, the absorbent article includes a pair of leak-barrier cuffs lying on the side of the barrier sheet facing a wearer's skin and opposed to each other in the transverse direction so as to extend in the front-to-rear direction, wherein the each of these leak-barrier cuffs comprises the fixed edge bonded to the chassis, the free edge not bonded to the chassis and being able to be spaced from the chassis and the cuff's elastic member attached under tension to the free edges and functioning to space the leak-barrier cuffs from the chassis, and the barrier sheet comprises the first rear anchoring zone in which the lateral zones are bonded to the chassis so as to extend in the front-to-rear direction aside from the middle zone toward the rear end and the second rear anchoring zone in which the lateral zones are bonded to the leak-barrier cuffs so as to extend in the front-to-rear direction aside from the middle zone toward the rear end wherein the first rear anchoring zone has a length dimension set to be longer than that of the second rear anchoring zone as measured in the front-to-rear direction.

### EFFECT OF THE INVENTION

According to the invention, the lateral zones of the barrier sheet are cut off partially and formed just outside of the middle zone as viewed in transverse direction with the notches so as to reduce the area over which the barrier sheet comes in contact with a wearer's skin. In this way, stuffiness and eczema due to the barrier sheet can be effectively alleviated.

According to the embodiment of the invention wherein the notches are formed about the border line and divided by the border line into the front and rear zones in the front-to-rear direction wherein the front zones has area larger than the rear region, eczema in a forward area of the diaper due to stuffiness, a possibility of which is higher than that in a rear forward area of the diaper, can be reduced.

According to the embodiment of the invention wherein there is provided between the barrier sheet and the chassis the partition sheet extending from the middle zone to the notches in the transverse direction adapted to partition the pocket into the sides of the front and rear region, urine and feces discharged in the pocket can be prevented from leaking out of the notches.

According to the embodiment of the invention wherein the barrier sheet is provided in the lateral zones thereof with the middle elastic members extending in the transverse direction, the area over which the barrier sheet comes to contact with the wearer's skin is sufficiently reduced to alleviate stuffiness and therefore eczema due to an excessive area.

According to the embodiment of the invention wherein the absorbent article includes the pair of leak-barrier cuffs lying on the side of the barrier sheet facing the wearer's skin and opposed to each other in the transverse direction so as to extend in the front-to-rear direction, and the barrier sheet comprises the first rear anchoring zone in which the lateral zones are bonded to the chassis so as to extend in the front-to-rear direction aside from the middle zone toward the rear end and the second anchoring zone in which the lateral zones are bonded to the leak-barrier cuffs so as to extend in the front-to-rear direction aside from the middle zone toward the rear end wherein the first rear anchoring zone has a length dimension set to be longer than that of the second rear anchoring zone, i.e., the second rear anchoring has a length dimension set to be shorter than that of the first rear anchoring zone. Therefore, the leak-barrier cuff and the barrier sheet are not bonded in relatively long zone so that the opening area of the rear through-hole would not excessively large, and consequently the rear through-hole would not allow any amount of feces to move back from the pocket.

### BRIEF DESCRIPTION OF THE DRAWINGS

[FIG. 1] Fig. 1 is a perspective view showing a diaper as a first embodiment according to the invention.
[FIG. 2] Fig. 2 is a sectional view taken along the II-II in Fig. 1.
[FIG. 3] Fig. 3 is a plan view showing the diaper of Fig. 1 as has been flatly developed.
[FIG. 4] Fig. 4 is a schematic diagram illustrating an important part as seen in Fig. 3.
[FIG. 5] Fig. 5 is a schematic diagram illustrating an important part as seen in Fig. 3.
[FIG. 6] Fig. 6 is schematic diagram illustrating an important part as seen Fig. 3.
[FIG. 7] Fig. 7 is a sectional view taken along the line VII-VII in Fig. 15.
[FIG. 8] Fig. 8 is a schematic diagram illustrating an important part in a second embodiment according to the invention.
[FIG. 9] Fig. 9 is a schematic diagram illustrating an important part in the third embodiment according to the invention.
[FIG. 10] Fig. 10 is a schematic diagram illustrating a middle elastic members in Fig. 9 now under contraction thereof.

### IDENTIFICATION OF REFERENCE NUMERALS USED IN THE DRAWINGS

- 1: diaper
- 2: chassis
- 3: barrier sheet
- 4: leak-barrier cuffs
- 5: side facing wearer's skin
- 6: side facing away from wearer's skin
- 7: front waist region
- 8: rear waist region
- 9: crotch region
- 10: liquid-absorbent structure
- 17: separator sheet
- 27: front end
- 28: rear end
- 29: pocket
- 30: lateral zone
- 31: lateral zone
- 32: middle zone
- 33: front through-hole
- 34: rear through-hole
- 39: barrier sheet's first elastic member
- 40: barrier sheet's second elastic member
- 41: notches
- 41a: notch's front half
- 41b: notch's rear half
- 42: side edge
- 43: side edge
- 48: cuffs' elastic members
- 51: first anchoring belt zone
- 52: second anchoring belt zone
- 53: first front anchoring belt zone
- 54: first rear anchoring belt zone
- 55: second front anchoring belt zone
- 56: second rear anchoring belt zone
- 57: notches
- 58: middle elastic members

### IDENTIFICATION OF REFERENCE NUMERALS USED IN THE DRAWINGS

The present invention will be exemplarily described hereinafter on the basis of a disposable diaper taken as typical embodiments of the absorbent article according to the present invention.

### <First embodiment of the invention>

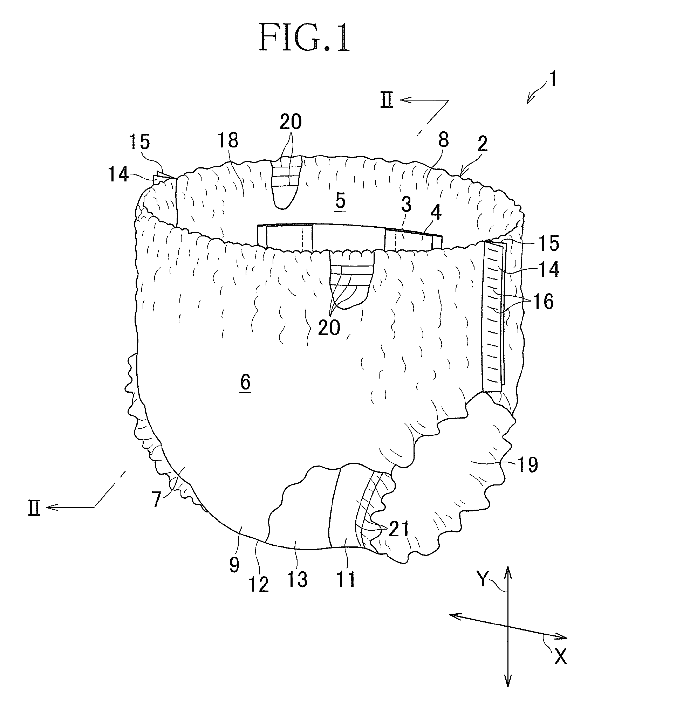

Figs. 1 through 7 illustrate a first embodiment according to the invention. Fig. 1 shows a diaper 1 as put on the wearer's body. As shown, the diaper 1 comprises a liquid-absorbent chassis 2, a barrier sheet 3 and leak-barrier cuffs 4. The chassis 2 is pants-shaped and defined by a transverse direction X, a vertical direction Y, an inner side 5 facing a wearer's skin, an outer side 6 facing the wearer's clothes, a front waist region 7, a rear waist region 8 and a crotch region 9 extending between said front and rear waist regions 7, 8. The chassis 2 comprises a topsheet 11, a backsheet 12 and a liquid-impervious leak-barrier sheet 13 sandwiched between these top- and backsheets 11, 12.

The front and rear waist regions 7, 8 respectively have pairs of side edges 14, 15 put flat and joined together at a plurality of joints 16 arranged intermittently along the respective side edges 14, 15 to form seams. In this way, the front and rear waist regions 7, 8 are joined together along the respective side edges 14, 15 so as to form a waist-opening 18 surrounded by the front and rear waist regions 7, 8 and a pair of leg-openings 9 surrounded by the respective rows of joints 16 and the crotch region 9. A plurality of waist elastic members 20 circumferentially extend along a peripheral edge of the waist-opening 18 and a plurality of leg elastic members 21 circumferentially extend along respective peripheral edges of the leg-openings 19. These elastic members 20, 21 are sandwiched between the topsheet 11 and the backsheet 12 and bonded under tension to at least one of these sheets 11, 12 by means of adhesive (not shown).

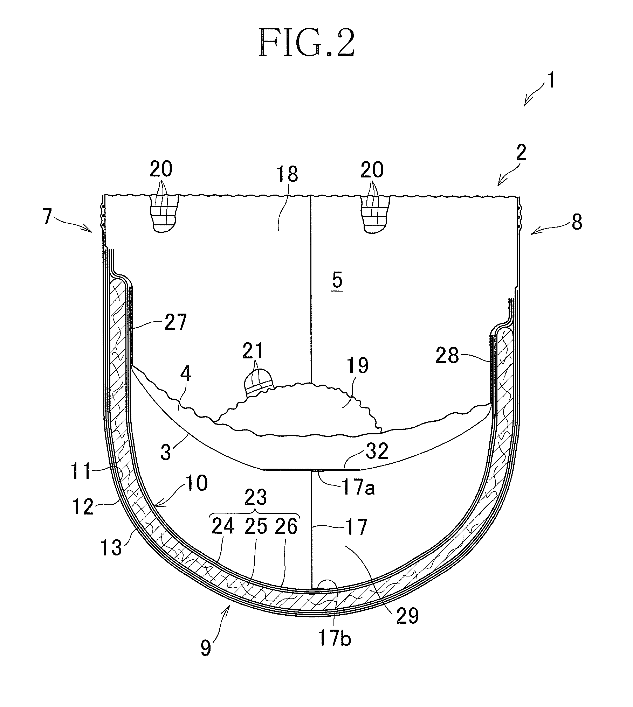

Fig. 2 is a sectional view taken along the line II-II in Fig. 1. As will be seen in Fig. 2, the liquid-absorbent structure 10 is disposed on the inner side 5 of the chassis 2 facing a wearer's skin, the barrier sheet 3 is disposed on the side of the liquid-absorbent structure 10 facing the wearer's skin, and the leak-barrier cuffs 4 is disposed on the side of the barrier sheet 3 facing the wearer's skin. While the liquid-absorbent structure 10 extends across the crotch region 9 and further into the front and rear waist regions 7, 8 in the in the longitudinal direction Y in this embodiment, the liquid-absorbent structure 10 will effectively function so far as it extends at least across the crotch region 9. The liquid-absorbent structure 10 includes a liquid-absorbent panel 123 disposed on the inner surface of the topsheet 11.

The liquid-absorbent panel 23 comprises a liquid-absorbent core 25 wrapped with a liquid-absorbent and -spreadable sheet 24 such as tissue paper and an inner sheet 26 covering the liquid-absorbent and -spreadable sheet 24. The side of the liquid-absorbent panel 23 facing wearer's clothes is covered with the leak-barrier sheet 13 which is disposed on the inner surface of the backsheet 12 to prevent bodily fluids once absorbed by the core 25 from leaking out from the diaper 1. The leak-barrier sheet 13 may be bonded directly to the bottom surface of the liquid-absorbent panel 23.

The barrier sheet 3 is bonded in the vicinity of its front and rear ends 27, 28 to the inner sheet 26 by means of adhesion or sealing. The front end 27 and the rear end 28 are located in the front waist region 7 and the rear waist region 8, respectively. In a pants-shaped state as illustrated, the crotch region 9 is bowed and the barrier sheet 3 is spaced upward from the inner sheet 26 in the vertical direction Y under contraction of barrier sheet's elastic members which will be described later. The front and rear ends 27, 28 of the barrier sheet 3 are bonded to the inner sheet 26 so that the barrier sheet 3 as a whole takes a posture like a hammock being slung. As a result, a pocket 29 is defined between the barrier sheet 3 and the liquid-absorbent panel 23.

Between the barrier sheet 3 and the liquid-absorbent structure 10, there is provided a partition sheet 17 adapted to partition the pocket 29 into the side of the front waist region 7 and the side of the rear waist region 8. The partition sheet
17 has upper and lower ends 17a, 17b opposed to each other in the longitudinal direction Y and extending in the transverse direction X wherein the upper end 17a is joined to the barrier sheet 3 by means of sealing or adhesion and the lower end 17b is joined to the inner sheet 26 by means of sealing or adhesion. The partition sheet 17 extends from the front side to the back side with respect to the sheet of drawing of Fig. 2 and raises itself on the inner sheet 26 to partition the pocket 29 back and forth as the barrier sheet 3 is spaced from the liquid-absorbent structure 10. This partition sheet 17 is formed by nonwoven fabric, plastic film or the like which is preferably liquid-impervious. The leak-barrier cuff 4 is spaced upward from the barrier sheet 3 under the effect of cuff's elastic member as will be described later so as to form a wall extending in the longitudinal direction Y.

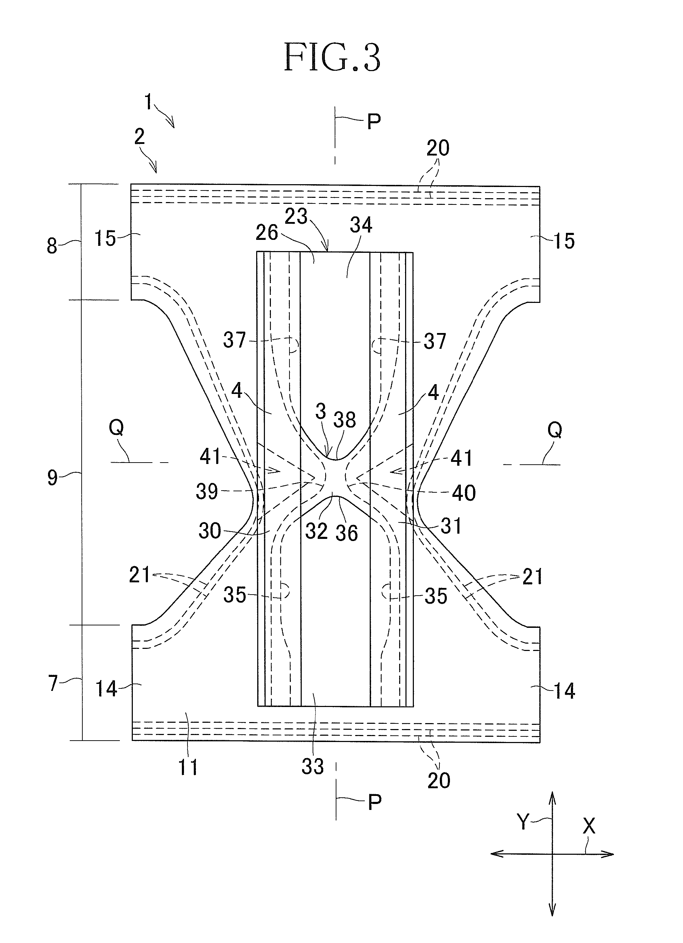

Fig. 3 is a plan view of the diaper 1 as flatly developed in the longitudinal direction Y as well as in the transverse direction X after the front and rear waist regions 7, 8 have been separated from each other along the respective rows of the joints 16 as seen in Fig. 1. In the state of the diaper 1 shown herein, the respective elastic members are under tension so as to keep the diaper 1 in the flattened state. As will be apparent from Fig. 3, the diaper 1 has a longitudinal center line P-P bisecting a dimension of the diaper 1 in the transverse direction X and a transverse center line Q-Q bisecting a dimension of the diaper 1 in the longitudinal direction Y and is bilaterally-symmetric about the longitudinal center line
P-P.

The chassis 2 has a concave shape curved inwardly and the liquid-absorbent panel 23 is rectangular and laminated on the topsheet 11 of the chassis 2. The barrier sheet 3 comprises a pair of lateral zones 30, 31 opposed to and spaced from each other in the transverse direction X and a middle zone 32 extending between these lateral zones 30, 31 wherein the middle zone 32 is located in the crotch region 9. The lateral zones 30, 31 cooperate with the middle zone 32 to form front and rear through-holes 33, 34. The front through-hole 33 has a substantially U-shape extending from the middle zone 32 into the front waist region 7 and the rear through-hole 34 has a substantially U-shape extending from the middle zone 32 into the rear waist region 8. The front through-hole 33 is defined by a pair of inner side edges 135 of the respective lateral zones 30, 31 and a curved margin of closure 36 connecting these inner side edges 35 to each other. The rear through-hole 34 is defined by a pair of inner side edges 37 of the respective lateral zones 30, 31 and a curved margin of closure 38 connecting these inner side edges 37 to each other.

The leak-barrier cuffs 4 comprise a pair of sheets extending in the longitudinal direction Y in symmetric relationship about the longitudinal center line P-P and formed, for example, by nonwoven fabric or plastic film which is preferably liquid-impervious. The leak-barrier cuffs 4 are formed so as to overlap respective skin-contacting surfaces of the lateral zones 30, 31 of the barrier sheet 3. Each of the leak-barrier cuffs 4 has a dimension in the transverse direction X substantially same as or larger than a dimension of the individual lateral zone 30, 31 of the barrier sheet 3 in the transverse direction X.

This diaper 1 should be put on a wearer's body with the wearer's external genital opposed to the front through-hole 33, the wearer's anus opposed to the rear trough-hole 34 and a zone of the wearer's skin defined between the external genital and the anus opposed to the middle zone 32. With the diaper 1 put on the wearer's body in this manner, the margin of closure 36 of the front through-hole 33 is preferably positioned aside forward from the transverse center Q-Q and the margin of closure 38 of the rear through-hole 34 is preferably positioned just on or in the vicinity of the transverse center line Q-Q. Thereupon, the liquid-absorbent panel 23 is bowed in the longitudinal direction Y and the barrier sheet 3 lifted up into contact with the wearer's skin under contraction of the barrier sheet's elastic members 39, 40 so that the pocket 29 (See Fig. 2) is formed between the barrier sheet 3 and the liquid-absorbent panel 23. In this way, body waste would be reliably received by the pocket 29 through the front and rear through-holes 33, 34 and thus prevented from coming in direct contact with a wearer's skin.

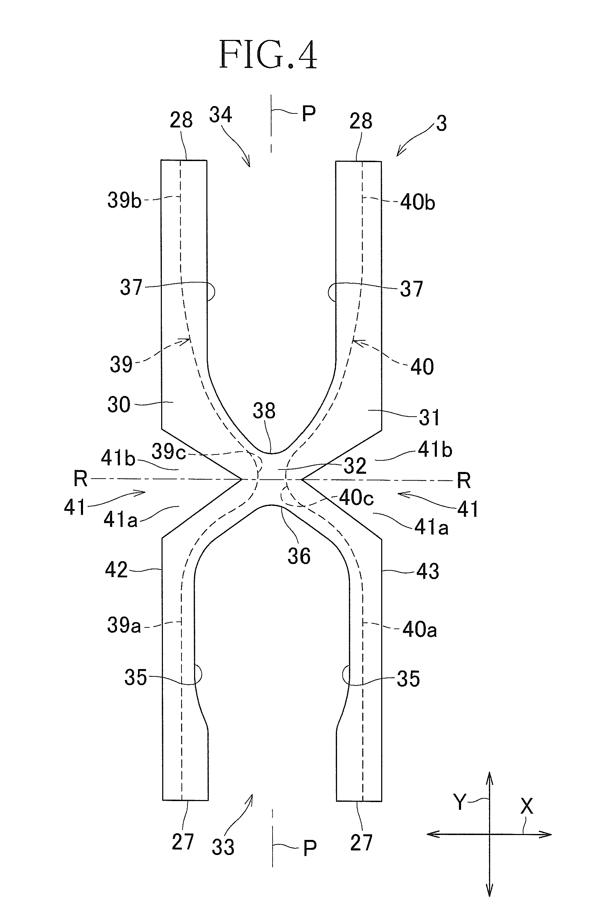

Fig. 4 illustrates details of the barrier sheet 3, wherein the other members are not illustrated. The front and rear through-holes 33, 34 have substantially U-shapes defined by substantially rectilinear inner side edges 35, 37 and the curved margins of closure 36, 38 and extending from the middle zone 32 toward the front and rear ends 27, 28. First and second barrier sheet's elastic members 39, 40 extending along the front and rear through-holes 33, 34 in the longitudinal direction Y are attached under tension to the barrier sheet 3.

The first and second barrier sheet's elastic members 39, 40 are symmetric about the longitudinal center line P-P and comprise front segments 39a, 40a extending along the front through-hole 33, rear segments 39b, 40b extending along the rear through-hole 34 and convex segments 39c, 40c extending toward the longitudinal center line P-P in the middle zone 32. A distance W1 in the transverse direction X between the first and second barrier sheet's elastic members 39, 40 is most small between the convex segments 39c, 40c and this distance in the transverse direction X between the front segments 39a and 40a as well as between the rear segments 39b, 40b is gradually enlarged from in the middle zone 32 toward the front and rear ends 27, 28.

The opposite lateral zones 30, 31 extending outside the middle zone 32 as viewed in the transverse direction are formed with notches 41. These notches 41 are formed by cutting the barrier sheet 3 so as to describe V-shapes respectively opening from points lying outside the convex segment 39c, 40c as viewed in the transverse direction X toward opposite side edges 42, 43 lying outside the respective lateral zones 30, 31 as viewed in the transverse direction X. To form these notches 41, the side edges 42, 43 also are partially cut off.

The middle zone 32 includes a border line R-R bisecting a dimension of the middle zone 32 in the longitudinal direction Y and the notches 41 are formed about this border line R-R. Each of the notches 41 comprises a front zone 41a extending from the border line R-R forward in the longitudinal direction Y and a rear zone 41b extending from the border line R-R rearward in the longitudinal direction Y. The front zone 41a is dimensioned to have an area larger than that of the rear zone 41b.

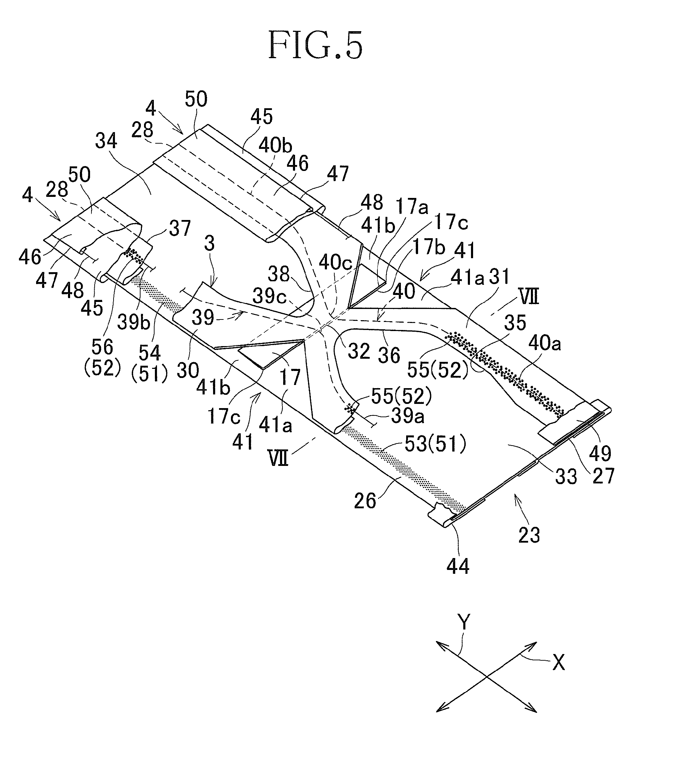

Fig. 5 is a diagram partially cutaway for convenience of illustration, showing details of the liquid-absorbent panel 23, the barrier sheet 3 and the leak-barrier cuffs 4. As shown, the barrier sheet 3 is placed on the side of the liquid-absorbent panel 23 facing a wearer's skin and the leak-barrier cuffs 4 are placed on the side of the barrier sheet 3 facing the wearer's skin.

The partition sheet 17 is sandwiched between the liquid-absorbent panel 23 and the barrier sheet 3. The partition sheet 17 is formed in the middle zone 32 wherein the upper end 17a is joined to the middle zone 32 and the lower end 17b is joined to the inner sheet 26 of the liquid-absorbent panel 23. A dimension of the partition sheet 17 in the transverse direction X is larger than that of the middle zone 32 and the partition sheet 17 has side edges 17c opposed in the transverse direction X extending beyond the middle zone 32 into the notches 41. The side edges 17c at the level of the upper end 17a are not bonded to the barrier sheet 3 while the side edges 17c at the level of the lower end 17b are bonded to the inner sheet 26. With the diaper put on a wearer's body, the barrier sheet 3 is spaced upward from the liquid-absorbent panel 23 and, in response to this, the partition sheet 17 raises itself in the direction in which the barrier sheet 3 is spaced from the liquid-absorbent panel 23. In consequence, the partition sheet 17 divides the pocket 29 back and forth. In the state of Fig. 5 showing the diaper before it is put on a wearer's body, the partition sheet 17 has its upper and lower ends 17a, 17b collapsed together.

The lateral zones 30, 31 of the barrier sheet 3 are folded back toward the liquid-absorbent panel 23 so as to define two-ply structures. Each of these two-ply lateral zones 30, 31 contains therein the first and second barrier sheet's elastic members 39, 40 bonded thereto by means of adhesive (not shown).

Each of the leak-barrier cuffs 4 comprises a three-ply sheet having a Z-shaped cross section. More specifically, the leak-barrier cuff 4 comprises a first layer 44, a second layer 45 and a third layer 46. The first layer 44 is sandwiched between the liquid-absorbent panel 123 and the chassis 2 and bonded to the topsheet 11 of the chassis 2. The second layer 45 is folded back from the first layer 44 onto the side of the barrier sheet 3 facing a wearer's skin so as to be placed upon the lateral zone 30 or 31. The third layer 46 is folded back onto the side of the second layer 45 facing the wearer's skin. In this manner, the leak-barrier cuff 4 is folded back toward a wearer's skin in the order of the first, second and third layers 44, 45, 46 wherein the first layer 44 and the second layer 45 are placed upon each other via the liquid-absorbent panel 23 and the second layer 45 is placed directly upon the third layer 46. The third layer 46 is folded back onto itself along a side edge 47 thereof so that a cuff's elastic member 48 may be attached thereto. The cuff's elastic member 48 is sandwiched between this two-ply side edge 47 and bonded under tension thereto so as to extend in the longitudinal direction Y between the front and rear ends 49, 50.

At front and rear ends 49, 50 of the leak-barrier cuff 4, the first layer 44 is bonded to the liquid-absorbent panel 23, the second layer 45 is bonded to the barrier sheet 3 and the third layer 46 is bonded to the second layer 45. Under contractile force of the cuff's elastic member 48 exerted on the leak-barrier cuff 4, the second layer 45 and the third layer 46 are unfolded and thereby spaced from the barrier sheet 3 so as to form a wall as seen in Fig. 2. Portions of the second layer 45 and the third layer 46 spaced from the barrier sheet 3, i.e. not bonded to the barrier sheet 3 are referred to herein as free edges.

The barrier sheet 3 has opposite lateral zones 30, 31 bonded to the topsheet 26 of the chassis 2 via a first anchoring zone 51 and bonded to the leak-barrier cuff 4 via a second anchoring zone 52. More specifically, the first anchoring zone 51 is formed by hot melt adhesive or the like and divided in the vicinity of a notch 41 in the longitudinal direction Y into a first front anchoring zone 53 and a first rear anchoring zone 54. The first front anchoring zone 53 extends from a point defined in the vicinity of a front zone 41a of the notch 41 to the front end 27 and the first rear anchoring zone 54 extends from a point defined in the vicinity of a rear zone 41b of the notch 41 to the rear end 28. In the course of forming the first front anchoring zone 53 and the first rear anchoring zone 54, it is desirable to prevent the adhesive from running over the notch 41.

The second anchoring zone 52 is formed by hot melt adhesive or the like and divided in the longitudinal direction Y. Specifically, the second anchoring zone 52 comprises a second front anchoring zone 55 extending from a point defined in the vicinity of a front zone 41a of the notch 41 to the front end 27 and a second rear anchoring zone 56 extending from a point defined in the vicinity of a rear zone 41b of the notch 41 to the rear end 28. In the course of forming the second front anchoring zone 55 and the second rear anchoring zone 56, it is desirable to prevent the adhesive from running over the notch 41. The second front anchoring zone 55 at least partially overlaps respective front zones 39a, 40a of the first and second barrier sheet's elastic members 39, 40 and the second rear anchoring zone 56 at least partially overlaps respective rear zones 39b, 40b of the first and second barrier sheet's elastic members 39, 40.

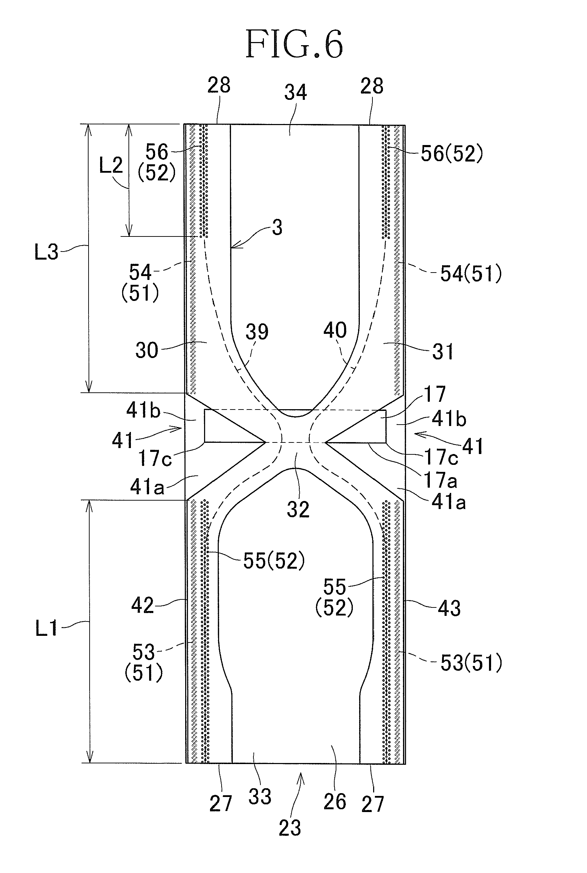

Fig. 6 is a diagram illustrating details of the barrier sheet 3, the liquid-absorbent panel 23, the first anchoring zone 51 and the second anchoring zone 52. As illustrated, the second front and rear anchoring zones 55, 56 are formed so as to lie inside the first front and rear anchoring zones 53, 54 as viewed in the transverse direction X. The first and second front anchoring zones 53, 55 have length dimensions L1 in the longitudinal direction Y which are substantially same. The second rear anchoring zone 56 has a length dimension L2 in the longitudinal direction Y which is shorter than a length dimension L3 of the first rear anchoring zone 54.

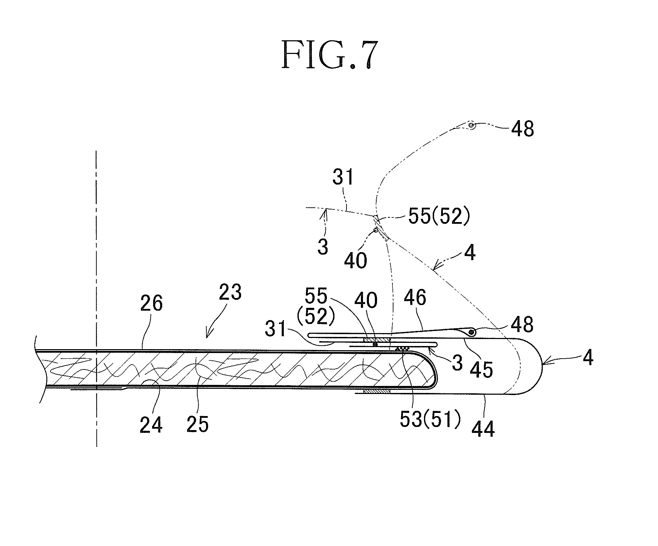

Fig. 7 is a sectional view taken along a line VII-VII in Fig. 5, wherein chain double-dashed lines indicate the first and second barrier sheet's elastic members 39, 40 and the cuff's elastic member 48 in respectively contracted states. While the lateral zone 31 of the barrier sheet 3 is illustrated herein, it will be appreciated that the lateral zone 30 is similar to the lateral zone 31 with respect to construction as well as function. As illustrated, the free edges of the second layer 45 and the third layer 46 of the leak-barrier cuff 4 are unfolded so as to be spaced from the liquid-absorbent panel 23 as the elastic members 40, 48 contract, i.e., as the diaper 1 is put on a wearer's body. With the leak-barrier cuff 4 being unfolded, the lateral zone 31 bonded to the liquid-absorbent panel 23 by the intermediary of the second anchoring zone 52 provided on the second layer 45 is spaced up- and outward in the transverse direction X from the liquid-absorbent panel 23. Consequentially it is ensured that the barrier sheet 3 can be reliably put into contact with a wearer's crotch region. Particularly because the second anchoring zone 52 is provided aside inward from the first anchoring zone 51, the barrier sheet 3 can be bonded to the barrier cuff 4 so as to be spaced farther from the liquid-absorbent panel 23 when the leak-barrier cuff 4 raises itself, and thereby the barrier sheet 3 can be further reliably put in contact with a wearer's skin.

Having ensured that the barrier sheet 3 is reliably kept in contact with a wearer's skin, the presence of the notches 41 advantageously reduces the area over which the barrier sheet
3 is contact with a wearer's skin. By reducing the contact area, stuffiness due to the contact is correspondingly alleviated and thereby wearers can be protected against suffering from eczema due to stuffiness. The contact area may be reduced to improve air-permeability of the barrier sheet 3 and such improvement further enhances the preventive effect against eczema. During use of the diaper, the front side of the barrier sheet 3 as viewed in the longitudinal direction Y is apt to come in contact with a wearer's skin closer than the rear side of the barrier sheet 3 and, on the front side, a possibility that the wearer may suffer from stuffiness due to urination is relatively high. In view of this, the front zone 41a of the notch 41 may be dimensioned to be larger than the rear zone 41b to further enhance the preventive effect against stuffiness due to contact of the barrier sheet 3 with a wearer's skin.

In the notches 41, a wearer's skin comes directly in contact with the inner sheet 26 of the liquid-absorbent structure 10 and therefore moisture such as wearer's sweat can be directly absorbed by the liquid-absorbent structure 10 to alleviate the wearer's eczema due to stuffiness. The side edges 17c of the partition sheet 17 are exposed in the respective notches 41. In the middle zone 32, the partition sheet 17 partitions the pocket 29 back and forth and thereby the partition sheet 17 prevents bodily fluids once urinated into the front through-hole 33 from moving to the rear through-hole 34 and prevents body waste once defecated in the rear through-hole 34 from moving to the front through-hole 33. In the notches 41, the upper end 17a of the partition sheet 17 is not bonded to the barrier sheet 3 and its side edges 17c are exposed. Therefore these portions of the partition sheet 17 serve to wrap around body waste such as feces tending to run off the notches 41 and to block it.

By bisecting the first and second anchoring zones 51, 52, respectively, in the longitudinal direction Y, coating of adhesive can be carried out passing over the respective notches 41. In this way, it is possible to prevent adhesive from coming in direct contact with a wearer's skin and thereby to protect the wearer's skin from any skin trouble due to contact with adhesive. The first and second anchoring zones 51, 52 are not bonded to each other so that the barrier sheet 3 can be pulled by the second front and rear anchoring zones 55, 56 in four directions, i.e., forward, rearward, leftward and rightward when the diaper is put on a wearer's body and the leak-barrier cuffs 104 raise themselves. In consequence, the barrier sheet 3 can be prevented from being slacked in the vicinity of the middle zone 32 and this middle zone 32 of the barrier sheet 3 can be reliably kept in contact with a wearer's skin.

The second front anchoring zones 55 are formed on the lateral zones 30, 31 of the front through-hole 33 and therefore the lateral zones 30, 31 can be pulled outward as viewed in the transverse direction X via these second front anchoring zones 55 as the leak-barrier cuffs 4 raise themselves. The opening area of the front through-hole 33 is broadened as the lateral zones 30, 31 are pulled outward in the transverse direction X. In view of the fact that the front through-hole 33 is adapted to be opposed to a wearer's external genital, its opening area may be broadened to prevent the barrier sheet 3 from coming in contact with the wearer's external genital and thereby to protect the wearer's skin against any excessive irritation.

The second rear anchoring zone 56 is dimensioned to be shorter than the first rear anchoring zone 54 in the longitudinal direction Y, so that the opening area of the rear through-hole 34 would not become excessively large. In view of the fact that the rear through-hole 34 having an excessively large opening area might allow any amount of feces to move back from the pocket, the rear through-hole 34 having the opening area properly limited according to this embodiment can prevent such undesirable phenomenon. The first rear anchoring zone 54 dimensioned to be longer than the second rear anchoring zone 56 in the longitudinal direction Y facilitates the lateral zones 30, 31 of the barrier sheet 3 to raise themselves like barriers. As a result, that any amount of feces would not flow back from the rear through-hole 34 to the liquid-absorbent panel 23 underlying the notches 41 and move back through the notches 41 in contact with the wearer's skin.

While the barrier sheet 3 is bonded to the chassis 2 by means of the first anchoring zones 51 and bonded to the leak-barrier cuffs 4 by means of the second anchoring zones 52 according to this embodiment, it is not essential to provide these first and second anchoring zones 51, 52 and the desired function may be achieved also by one of them. The first and second anchoring zones 51, 52 may be formed by rubber-based hot melt adhesive conventionally used in the field of art or by conventionally used technique such as sonic sealing or heat sealing. Furthermore, the first and second anchoring zones 51, 52 may be formed so as to be continuously anchored or intermittently anchored on the barrier sheet 3, in each case, so as to describe a belt-like shape.

While the barrier sheet 3 and the leak-barrier cuff 4 respectively comprise single sheets according to this embodiment, those also may be formed by adhesively bonding a pair of sheets, respectively. In this case, the first and second barrier sheet's elastic members 39, 40 as well as the cuff's elastic member 48 may be sandwiched between a pair of sheets, respectively. When the barrier sheet 3 is formed by a pair of sheets, peripheral edges of these sheets defining the notch 41 are preferably bonded by adhesion or sealing to prevent body waste from intruding through a gap otherwise defined between the peripheral edges of the sheets. While the barrier sheet 3 has the U-shaped front and rear through-holes 33, 34 according to this embodiment, the invention is not limited to this and the barrier sheet 3 may have O-shaped front and rear through-holes cut away therefrom.

According to the second aspect of the invention, the barrier sheet 3 as well as the leak-barrier cuffs 4 may be formed, for example, by liquid-impervious and air-permeable nonwoven fabric, the topsheet 11 as well as the backsheet 12 may be formed, for example, by air-permeable nonwoven fabric, the leak-barrier sheet 13 may be formed, for example, by plastic film and the liquid-absorbent core 25 may be formed, for example, by a mixture of fluff pulp and super-absorbent polymer particles. All of these materials have conventionally been used in the related field of technique.

### <Second embodiment according to the invention>

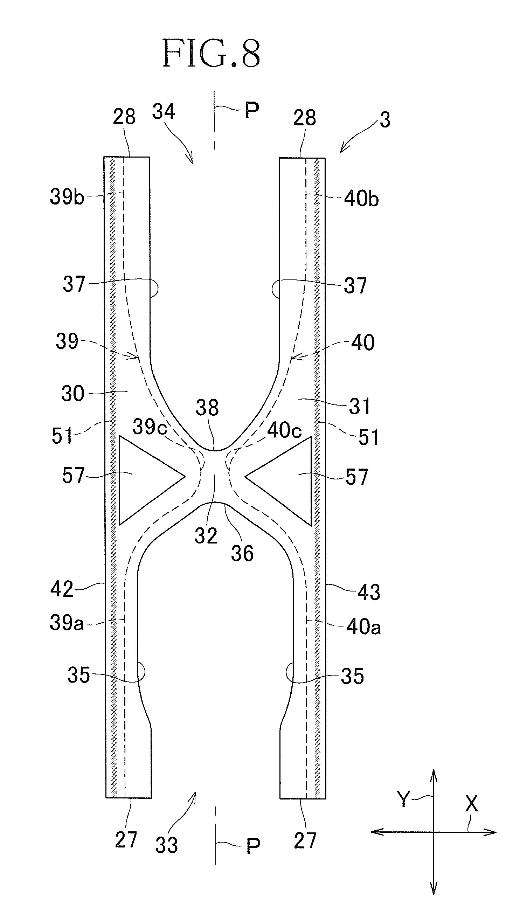

Fig. 8 illustrates a second embodiment according to the invention. According to this second embodiment, the notch formed in the barrier sheet 3 has s configuration distinguished from that in the first embodiment but the other components are similar to those in the first embodiment. Therefore, no detailed description of these similar components will be repeated.

According to this embodiment, the lateral zones 30, 31 of the barrier sheet 3 are partially cut out to form the notches 57. These notches 57 respectively open from points lying outside the convex segments 39c, 40c of the first and second barrier sheet's elastic members 39, 40 as viewed in the transverse direction X toward the side edges 42, 43, respectively. It should be noted here that these notches are formed by cut out the barrier sheet 03 without partially cutting off the side edges 42, 43 so as to define substantially triangular notches rather than cut off the barrier sheet 3. By forming the barrier sheet 3 with the notches 57 in this manner, the area of the barrier sheet 3 coming in contact with a wearer's skin can be reduced and thereby the wearer's skin would not suffer from eczema.

According to this embodiment, portions of the barrier sheet 3 remain intact along the side edges 42, 43 and therefore it is possible to form the first anchoring zone 51 serving to bond the barrier sheet 3 and the inner sheet 26 together without dividing the first anchoring zone 51 in two. However, it will be appreciated that the first anchoring zone 51 is preferably divided back and forth if it is desired to enlarge a distance between the barrier sheet 3 and the chassis when the barrier sheet 3 is spaced upward from the chassis.

It is also possible to form the lateral zones 30, 31 with a plurality of small through-holes to reduce the contact area.

### <Third embodiment according to the invention>

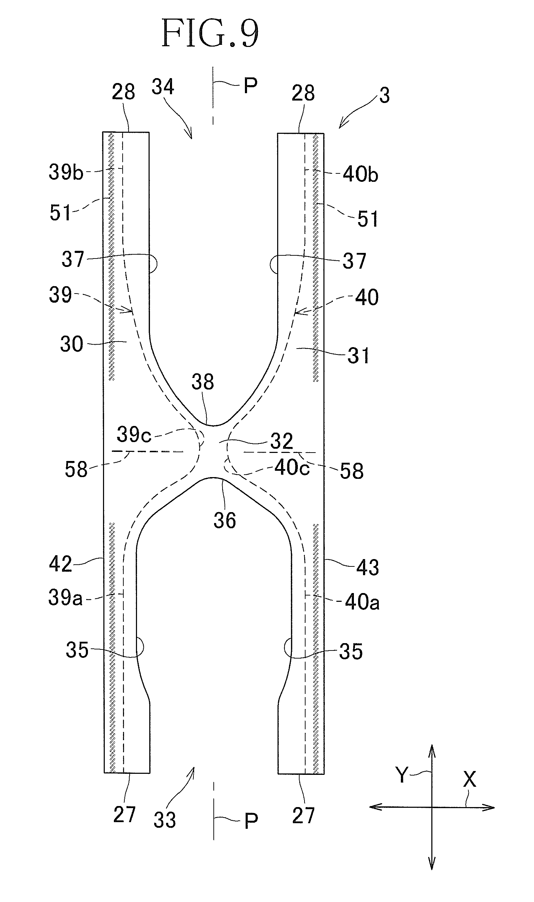

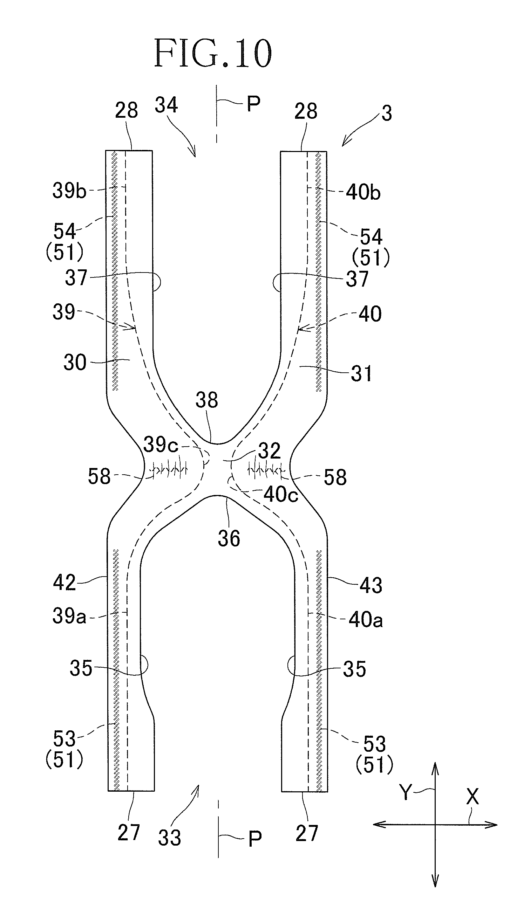

Figs. 9 and 10 illustrate a third embodiment according to the invention **characterized in that** the barrier sheet 3 is provided in the lateral zones 30, 31 with middle elastic members 58, respectively. The other features are similar to those in the first embodiment and details of them will not be described repetitively.

The middle elastic members 58 are provided in the middle zone 32 outside the convex segments 39c, 40c of the first and second barrier sheet's elastic members 39, 40 as viewed in the transverse direction X. These middle elastic members 58 extend outward as viewed in the transverse direction X from points lying in the vicinity of the respective convex segments 39c, 40c toward the side edges 42, 43 and bonded under tension to the barrier sheet 3. Outside the middle elastic members 58 in the transverse direction X, each of the first anchoring zones 51 is divided into first front and rear anchoring zones 53, 54. Specifically, outside each of the middle elastic members 58 in the transverse direction X, the barrier sheet 3 is bonded neither to the inner sheet 26 nor to the leak-barrier cuff 4.

Under contraction of the middle elastic members 58 during use of the diaper, the lateral zones 30, 31 of the barrier sheet 3 are pulled inward in the transverse direction X toward the longitudinal center line P-P and shrunk in this direction, as shown in Fig. 10. Consequently, the area over which the barrier sheet 3 comes in contact with a wearer's skin is sufficiently reduced to alleviate stuffiness and therefore eczema due to an excessive contact area.

## Claims

1. An absorbent article comprising a chassis (2) having a front-to-rear direction and a transverse direction, sides facing a wearer's skin and wearer's clothes (5, 6), respectively, a front waist region (7), a rear waist region (8) and a crotch region (9) extending between said front and rear waist regions, and a liquid-absorbent structure (10) disposed on said crotch region, and a barrier sheet (3) lying on said side of said chassis facing a wearer's skin and provided in said crotch region so as to be spaced from said chassis, said absorbent article being **characterized in that:**
said barrier sheet has a pair of lateral zones (30, 31) extending in said front-to-rear direction and opposed to each other in said transverse direction, a middle zone (32) connecting said lateral zones to each other, front and rear ends (27, 28) extending in said transverse direction and opposed to each other in said front-to-rear direction and barrier sheet's elastic members (39, 40) extending in said front-to-rear direction and attached under tension to said barrier sheet and opposed to each other in said transverse direction wherein said front and rear ends are permanently bonded to the side of said chassis facing a wearer's skin and said liquid-absorbent structure is adapted to be bowed in a direction from said crotch region toward said front and rear waist regions and thereby to be spaced from said chassis and form a pocket (29) between said barrier sheet and said liquid-absorbent structure;
through-holes (33, 34) are defined by said pair of lateral zones and said middle zone so that body waste can pass said through-holes into said pocket; and
said pair of lateral zones lying outside said middle zones as viewed in said transverse direction are partially cutaway to form notches (41).

2. The article according to Claim 1, wherein said notches are formed by cutting off said lateral zones partially inclusive of side edges wherein said side edges are forming the outside of said pair of lateral zones as viewed in transverse direction.

3. The article according to Claim 1, wherein said notches are formed by cutting out said lateral zones with side edges remaining wherein said side edges are forming outside of said pair of lateral zones as viewed in transverse direction.

4. The article according to any one of Claims 1 through 3, wherein said through-hole comprises a front through-hole lying on said front side with respect to said middle zone in said front-to-rear direction and a rear through-hole lying on the rear side with respect to said middle in said front-to-rear direction, said barrier sheet includes a border line bisecting said middle zone in said front-to-rear direction, said notches are formed about said border line and divided by said border line into front and rear zones in said front-to-rear direction wherein said front zone has an area larger than said rear zone.

5. The article according to any one of Claims 1 through 4, there is provided between said barrier sheet and the chassis a partition sheet extending from said middle zone to said notches in said transverse direction and adapted to partition the pocket into said side of the front waist region and said side of said rear waist region.

6. An absorbent article comprising a chassis (2) having a front-to-rear direction and a transverse direction, sides facing a wearer' s skin and wearer' s clothes (5, 6), respectively, a front waist region (7), a rear waist region (8) and a crotch region (9) extending between said front and rear waist regions, and a liquid-absorbent structure (10) disposed on said crotch region, and a barrier sheet (3) lying on said side of said chassis facing a wearer's skin and provided in said crotch region so as to be spaced from said chassis, said absorbent article being **characterized in that:**
said barrier sheet has a pair of lateral zones extending in a front-to-rear direction and opposed to each other in a transverse direction, a middle zone connecting the lateral zones to each other, front and rear ends extending in said transverse direction and opposed to each other in said front-to-rear direction and barrier sheet's elastic members extending in said front-to-rear direction and attached under tension to said barrier sheet and opposed to each other in said transverse direction wherein said front and rear ends are permanently bonded to the side of said chassis facing a wearer's skin and said liquid-absorbent structure is adapted to be bowed in a direction from said crotch region toward said front and rear waist regions and thereby to be spaced from said chassis and form a pocket between said barrier sheet and said liquid-absorbent structure, through-holes are defined by said pair of lateral zones and said middle zone so that body waste can pass through-holes into the pocket, said lateral zones lying outside said middle zones as viewed in said transverse direction are provided with middle elastic members attached under tension thereto so as to extend in said transverse direction wherein, outside of these middle elastic members as viewed in said transverse direction, said barrier sheet and said chassis are not bonded to each other.

7. The article according to any one of Claims 1 through 6, wherein said absorbent article includes a pair of leak-barrier cuffs lying on said side of the barrier sheet facing a wearer's skin and opposed to each other in the transverse direction so as to extend in the front-to-rear direction, wherein the each of these leak-barrier cuffs comprises said fixed edge bonded to said chassis, the free edge not bonded to said chassis and being able to be spaced from said chassis and said cuff' s elastic member attached under tension to said free edges and functioning to space said leak-barrier cuffs from said chassis, and said barrier sheet comprises said first rear anchoring zone in which said lateral zones are bonded to said chassis so as to extend in said front-to-rear direction aside from said middle zone toward said rear end and said second rear anchoring zone in which said lateral zones are bonded to said leak-barrier cuffs so as to extend in said front-to-rear direction aside from said middle zone toward said rear end wherein said first rear anchoring zone has a length dimension set to be longer than that of said second rear anchoring zone as measured in said front-to-rear direction.
